# EUROPÄISCHE PATENTSCHRIFT

(11) **EP 1 095 659 B1**
(45) Veröffentlichungstag und Bekanntmachung des Hinweises auf die Patenterteilung: **08.09.2004**
(21) Anmeldenummer: 00121935.1
(22) Anmeldetag: 09.10.2000
(51) Int. Cl.: A61K 35/78, A61K 47/38

(54) **Celluloseether stabilisierte Öl-in-Wasser Emulsionen als Träger für homöopathische und pflanzliche Wirkstoffe**
Oil-in-water emulsions stabilised by cellulose ethers as carriers for homeopathic and plant drugs
Emulsions huile-dans-eau stabilisées par des éthers cellulosiques comme véhicules de principes actifs homéopathiques et végétaux

(30) Priorität: 26.10.1999 DE 19951474
(43) Veröffentlichungstag der Anmeldung: 02.05.2001
(73) Patentinhaber: Dr. Willmar Schwabe GmbH & Co., 76227 Karlsruhe (DE)
(72) Erfinder: Wollenweber, Christian, 76228 Karlsruhe (DE); Oschmann, Rainer, Dr., 76829 Landau (DE); Heger, Marianne, Dr. med., 76698 Ubstadt-Weiher (DE); Daniels, R., Prof. Dr., 38226 Salzgitter (DE)
(74) Vertreter: Rudolph, Ulrike, Dr.

(56) Entgegenhaltungen:
- EP-A- 0 487 404
- WO-A-96/29056
- WO-A-99/22716

## Beschreibung

Die Erfindung betrifft eine Öl-in-Wasser Emulsionszubereitung mit einem Gehalt an einer alkoholhaltigen homöopathischen und/oder pflanzlichen Wirkstoffzubereitung.

Zubereitungen zum Auftragen auf die Haut (Dermatika) mit homöopathischen oder pflanzlichen Wirkstoffen sollten sich dadurch auszeichnen, dass sie nur eine geringe Anzahl Hilfsstoffe enthalten, die möglichst natürlichen Ursprungs sind. Auf einen Zusatz von Stabilisatoren, Antioxidantien und Konservierungsmittel sollte verzichtet werden. Die Herstellung und Zusammensetzung solcher Zubereitungen ist im Homöopathischen Arzneibuch (HAB) detailiert beschrieben. Für Salben ist dort beispielsweise vorgegeben (Vorschrift 13), daß wasserhaltige Wollwachsalkoholsalbe gemäß dem Deutschen Arzneibuch (DAB) als Grundlage, d.h. als Träger für den/die Wirkstoff(e) zu verwenden ist. Werden andere Grundlagen verwendet, so müssen diese deklariert werden. In diese Grundlage wird der bzw. werden die homöopathische(n) Wirkstoff(e) eingearbeitet. Solche Wasser-in-Öl- oder Öl - in - Wasser Emulsionszubereitungen, im folgenden abgekürzt zu "W/O-" bzw. "O/W-Emulsionszubereitungen", sind jedoch in den Fällen, in denen die homöopathischen Wirkstoffe in Form von Urtinkturen und/oder Dilutionen zugegeben werden, aufgrund des Alkoholgehaltes (insbesondere Ethanolgehaltes) dieser Urtinkturen/Dilutionen oftmals physikalisch wenig stabil. Daher besteht Bedarf an anderen Zubereitungen, die eine ausreichend hohe Alkohol-, insbesondere Ethanoltoleranz aufweisen, und die nach dem HAB zulässig sind.

Üblicherweise werden zur Stabilisierung von O/W-Emulsionszubereitungen Emulgatoren verwendet, beispielsweise in der EP 0 487 404 Al . Diese meist niedermolekularen Substanzen können jedoch aufgrund ihres amphiphilen Aufbaues Wechselwirkungen mit der Haut eingehen **[Ashton P., et al. ,** Effects of surfactants in percutaneous adsorption, Pharm. Acta Helv. 61 (8), 228-235, 1986] und so Unverträglichkeitsreaktionen wie z.B. toxisch-irritative Reaktionen oder Kontaktallergien hervorrufen **[Raab W., Kindl U.,** Unerwünschte Wirkungen von Kosmetika, in: Pflegekosmetik, Gustav Fischer Verlag, Stuttgart, 75-86, 1991].

Als geeignete Hilfsstoffe zur Emulsionsstabilisierung kommen nach dem Stand der Technik insbesondere auch Celluloseether, sogenannte Polymeremulgatoren, in Betracht. Sie eignen sich zur Emulsionsstabilisierung, da sie an O/W-Grenzflächen adsorbieren, die Grenzflächenspannung zwischen äußerer und innerer Phase senken und stabile Grenzflächenfilme ausbilden **[Daniels R.,** Hydrophile Polymere als Stabilisatoren für tensidfreie Öl-in-Wasser-Emulsionen, Habilitationsschrift Regensburg, 1994]. Celluloseether sind als Hilfsstoffe in der Pharmazie und Lebensmitteltechnologie weit verbreitet. In festen Arzneiformen finden sie als Bindemittel für Granulate, als Matrixbildner zur verzögerten Wirkstofffreisetzung sowie als magen- und darmsaftlöslicher Filmbildner Verwendung. In flüssigen Zubereitungen dienen sie vor allem zur Viskositätserhöhung von Augentropfen, in höherer Konzentration auch als Hydrogelbildner [Shin Etsu, Metolose, Water-soluble cellulose ether, Shin Etsu Chemical Co. Ltd, Tokyo, 1995]. Bei der Anwendung celluloseetherhaltiger Zubereitungen am Auge wurde eine gute Verträglichkeit festgestellt [**Kegel S.,** Besonderheiten der Prüfung viskoelastischer Materialien auf ihre Bioverträglichkeit an Zellkulturen: Beispiel Hydroxypropylmethylcellulose, Dissertation, Universität Aachen, 1994]. Unerwünschte Nebenwirkungen auf der Haut sind von dieser Substanzklasse nicht bekannt.
Die Verwendung von verschiedenen Celluloseethern zur Stabilisierung von alkoholfreien O/W-Emulsionen wurde in den letzten Jahren bereits untersucht **[Barta A.,** Herstellung und Bewertung von O/W Emulsionen unter der Verwendung von Celluloseethern als Polymeremulgatoren, Dissertation, Universität Regensburg, 1992], wobei insbesondere gute Ergebnisse mit Hypromellose-stabilisierten O/W-Emulsionen, die als Ölphase mittelkettige Triglyceride enthielten, erzielt wurden **[Rimpler S.,** Pharmazeutischtechnologische Charakterisierung von O/W-Emulsionen mit Methylhydroxypropylcellulose als Polymeremulgator, Dissertation, Universität Regensburg, 1996].

Neben dem Problem der befriedigenden Stabilisierung besteht bei den O/W-Emulsionszubereitungen außerdem das Problem, dass wasserhaltige Zubereitungen zum mikrobiellen Befall neigen: Keime können einerseits während der Herstellung und andererseits auch während der Anwendung durch den Patienten eingebracht werden **[Wallhäußer K. H.,** Die mikrobielle Reinheit von Arzneimitteln in der Hand des Verbrauchers, Deutsche Apothekerzeitung 41, 1510-1540, 1978]. Der Einsatz von Konservierungsmitteln in solchen Zubereitungen ist daher angezeigt, aber auch Konservierungsmittel gelten als Auslöser von Unverträglichkeitsreaktionen beim Patienten **[Raab W., Kindl U.,** Unerwünschte Wirkungen von Kosmetika, in: Pflegekosmetik, Gustav Fischer Verlag, Stuttgart, 75-86, 1991] und sollten aus diesem Grund wiederum eher vermieden werden.

Aufgabe vorliegender Erfindung ist es, gut verträgliche und ausreichend konservierte Emulsionszubereitungen als Grundlage für homöopathische Urtinkturen und Verdünnungen, flüssige Pflanzenextrakte sowie ethanolische Tinkturen bereitzustellen. Die Grundlagen sollten aus wenigen, wenn möglich in den Arzneibüchern (Pharmakopoia Europäa Ph Eur, Deutsches Arzneibuch DAB, Homöopathisches Arzneibuch HAB, United States Pharmacopoia USP) in Form einer Monografie beschriebenen und damit als unbedenklich einzustufenden Hilfsstoffen bestehen, und ihre Herstellung sowie die Einarbeitung des/der Wirkstoff(e) sollte in einer einfachen Art und Weise sowohl im Labor- als auch im Produktionsmaßtab möglich sein.

Eine Lösung dieser Aufgabe besteht in der Bereitstellung einer O/W-Emulsionszubereitung mit einem Gehalt an einer alkoholhaltigen homöopathischen und/oder pflanzlichen Wirkstoffzubereitung, die dadurch charakterisiert ist, dass die Emulsionszubereitung aus den Komponenten Öl, Wasser, Alkohol, homöopathischer und/oder pflanzlicher Wirkstoff und Celluloseether besteht.
Die erfindungsgemäßen celluloseetherstabilisierten, Wirkstoff-in-Alkohol-haltigen ( d.h.: Alkohol-und-homöopathische(n)-und-pflanzliche(n)- Wirkstoff(e)-haltigen) Öl-inWasser-Emulsionszubereitungen zeichnen sich vor allem dadurch aus, dass sie eine überraschend hohe Lagerstabilität aufweisen. Diese Lagerstabilität ist deutlich höher, als aufgrund des im Stand der Technik bekannten Fachwissens über Hypromellose-stabilisierte Emulsionen zu erwarten war. Die Erfindung beruht auf der überraschenden Erkenntnis, dass durch Alkoholzusatz und insbesondere Ethanolszusatz die Lagerstabilität von Hypromellose - stabilisierten Emulsionen wesentlich verbessert wird. Diese Erkenntnis steht im Kontrast zur bislang herrschenden Überzeugung der Fachwelt, gemäß der O/W-Emulsionen durch die Beimischung von alkoholhaltigen Flüssigkeiten wie beispielsweise flüssigen homöopathischen Tinkturen und Dilutionen oder alkoholischen Pflanzenextrakten instabil werden.
Außerdem haben die erfindungsgemäßen O/W-Emulsionszubereitungen den Vorteil, dass bei entsprechend hoher Alkoholkonzentration (insbesondere Ethanolkonzentration) von beispielsweise 15 Masseprozent (% m/m) die Emulsion ausreichend konserviert ist, so dass auf den Zusatz von anderen Konservierungsmitteln verzichtet werden kann.
Ein weiterer Vorteil der erfindungsgemäßen O/W-Emulsionszubereitungen besteht darin, dass die Herstellung dieser Emulsionen im Vergleich zur Herstellung herkömmlicher, alkohol- bzw. ethanolfreier Emulsionen wesentlich vereinfacht ist, weil infolge des Zusatzes der alkohol- bzw. ethanolhaltigen Wirkstoffzubereitungen und/oder des Zusatzes von reinem Ethanol eine Dispergierung der Polymeremulgatoren ohne Erwärmung auf hohe Temperaturen möglich ist.
Eine Lösung der der Erfindung zugrunde liegenden Aufgabe besteht folglich auch in der Bereitstellung eines Verfahrens zur Herstellung einer celluloseetherhaltigen O/W-Emulsionszubereitung mit einem Gehalt an homöopathischen und/oder pflanzlichen Wirkstoffen in Alkohol, das durch die Anzahl und Reihenfolge der nachstehend beschriebenen Verfahrensschritte gekennzeichnet ist:
1. Der bzw. die Celluloseether wird/werden in der alkoholisch-wässrigen Emulsionsphase gelöst, und
2. die so erhaltene celluloseetherhaltige alkoholisch-wässrige Emulsionsphase wird dann mit dem Öl (der Ölphase) homogenisiert.

Dieses erfindungsgemäße Herstellungsverfahren hat gegenüber den herkömmlichen Verfahren die Vorteile, dass es wesentlich schneller durchgeführt werden kann, da nur zwei Verfahrensschritte notwendig sind, während das herkömmliche Verfahren vier Schritte umfaßt, und dass zu seiner Durchführung weniger Energie aufgewendet werden muß, weil die Erwärmung der Emulsion zum Zwecke der Dispersion der Polymeremulgatoren nicht mehr erforderlich ist.

Zur Herstellung der erfindungsgemäßen celluloseetherstabilisierten , wirkstoffhaltigen Emulsionszubereitungen werden vorzugsweise die folgenden Celluloseether, einzeln oder in Kombination, und vorzugsweise in einer Menge von 0,1 bis 10 Masseprozent (bezogen auf die Gesamtmasse der Emulsionszubereitung) eingesetzt:
- Methylcellulose Ph Eur
- Hydroxypropylcellulose Ph Eur
- Hypromellose bzw. Methylhydroxypropylcellulose Ph Eur
- Methylhydroxyethylcellulose Ph Eur
- Hydroxyethyllcellulose Ph Eur
- Natriumcarboxymethylcellulose Ph Eur.

Als disperse Phase der erfindungsgemäßen O/W-Emulsionszubereitungen eignen sich insbesondere die folgenden Öle, einzeln oder in Kombination, und vorzugsweise in einer Menge von 1 bis 74 Masseprozent (bezogen auf die Gesamtmasse der Emulsionszubereitung):
- Mittelkettige Triglyceride Ph Eur
- Erdnußöl Ph Eur
- Rizinusöl Ph Eur
- dünnflüssiges Paraffin Ph Eur
- dickflüssiges Paraffin Ph Eur.

Als Wirkstoffe der erfindungsgemäßen Emulsionszubereitungen kommen homöopathische Urtinkturen und Dilutionen, flüssige alkoholische Pflanzenextrakte sowie sonstige alkoholische und insbesondere ethanolische Tinkturen in Betracht. Sie werden vorzugsweise in einer Menge von 1 bis 50 Masseprozent (bezogen auf die Gesamtmasse der Emulsionszubereitung) eingesetzt.

Um die Lagerstabilität und die Widerstandsfähigkeit gegen Keimbefall weiter zu erhöhen, können die erfindungsgemäßen Emulsionszubereitungen zusätzlich zu den alkoholischen Wirkstoffen einen Zusatz Ethanol, vorzugsweise in einer Menge von 1 bis 20 Masseprozent (bezogen auf die Gesamtmasse der Emulsionszubereitung) aufweisen. Sämtliche erfindungsgemäßen Emulsionszubereitungen eignen sich sehr gut zur Herstellung von homöopathischen und/oder phytopharmazeutischen Arzneimitteln, weshalb die vorliegende erfindungsgemäß auch solche homöopathischen und/oder phytopharmazeutischen Arzneimitteln, die unter Verwendung einer erfindungsgemäßen Emulsionszubereitung hergestellt worden sind, umfaßt.

Die Erfindung wird im folgenden anhand von Ausführungsbeispielen näher erläutert:

### Beispiel 1: Vergleich der Herstellung ethanolfreier (herkömmlicher) und ethanolhaltiger (erfindungsgemäßer) O/W Emulsionen

### (A) Ethanolfreie Hypromellose stabilisierte O/W-Emulsionen (Heissherstellung, herkömmliches Verfahren nach dem Stand der Technik)

1. Hypromellose wird in heissem Wasser (90 °C) dispergiert. Die Suspension wird unter Rühren auf Raumtemperatur abgekühlt. Es entsteht eine klare, opaleszierende Lösung. Verdunstetes Wasser wird ergänzt.
2. Urtinktur (bzw. Extrakt) wird der Hypromelloselösung unter Rühren zugegeben, bis eine homogene Lösung entsteht.
3. Wässrige Phase und Ölphase werden auf 40 °C erwärmt.
4. Die Ölphase wird in die wässrige Phase eingearbeitet. Die Homogenisierung erfolgt mit einem Rotor-Stator-Homogenisator. Es entsteht eine homogene Emulsion.

### (B) Ethanolhaltige Hypromellose stabilisierte O/W-Emulsionen (Kaltherstellung, erfindungsgemäßes Verfahren)

1. Hypromellose wird mit Ethanol angeteigt. Der Hypromellosesuspension werden Wasser und Urtinktur (bzw. Extrakt) unter Rühren zugegeben, bis eine homogene Lösung (wässrige Phase) entsteht.
2. Die Ölphase wird in die wässrige Phase eingearbeitet. Die Homogenisierung erfolgt mit einem Rotor-Stator-Homogenisator. Es entsteht eine homogene Emulsion.

### Beispiel 2: Rezeptur einer erfindungsgemäßen celluloseetherstabilisierten homöopathischen, urtinkturhaltigen O/W-Emulsion

### Inhaltsstoffe:

| | |
|---|---|
| Hypromellose Ph Eur | 1,0 % m/m |
| Cardiospermum halicacabum Ø HAB | 10,0 % m/m |
| Ethanol Ph Eur | 8,0 % m/m |
| Mittelkettige Triglyceride Ph Eur | 10,0 % m/m |
| Ger. Wasser Ph Eur | ad 100,0 % m/m |

### Zubereitung:

1. Die Hypromellose wird mit Ethanol angeteigt. Dieser Hypromellosesuspension werden Wasser und Cardiospermum halicacabum Urtinktur (bzw. Extrakt) unter Rühren zugegeben, bis eine homogene Lösung (wässrige Phase) entsteht.
2. Die mittelkettigen Triglyceride (Ölphase), werden in die wässrige Phase eingearbeitet, und diese Mischung wird mit einem Rotor-Stator-Homogenisator homogenisiert.

### Beispiel 3: Rezeptur einer erfindungsgemäßen celluloseetherstabilisierten Arnikatinkturhaltige O/W-Emulsion

### Inhaltsstoffe:

| | |
|---|---|
| Hypromellose Ph Eur | 2,0 % m/m |
| Arnikatinktur DAB | 50,0 % m/m |
| Ethanol Ph Eur | 5,0 % m/m |
| Mittelkettige Triglyceride Ph Eur | 5,0 % m/m |
| Ger. Wasser Ph Eur | ad 100,0 % m/m |

### Zubereitung:

1. Die Hypromellose wird mit Ethanol angeteigt. Dieser Hypromellosesuspension werden Wasser und Arnikatinktur Urtinktur (bzw. Extrakt) unter Rühren zugegeben, bis eine homogene Lösung (wässrige Phase) entsteht.
2. Die mittelkettigen Triglyceride (Ölphase), werden in die wässrige Phase eingearbeitet, und diese Mischung wird mit einem Rotor-Stator-Homogenisator homogenisiert.

### Beispiel 4: Rezeptur einer zweiten erfindungsgemäßen celluloseetherstabilisierten Cardiospermum halicacabum haltigen O/W-Emulsion

### Inhaltsstoffe:

| | |
|---|---|
| Natrium Carboxymethylcellulose Ph Eur | 3,0 % m/m |
| Cardiospermum halicacabum Ø HAB | 30,0 % m/m |
| Ethanol Ph Eur | 5,0 % m/m |
| Dünnflüssiges Paraffin Ph Eur | 20,0 % m/m |
| Gereinigtes Wasser Ph Eur | ad 100,0 % m/m |

### Zubereitung:

1. Die Natrium Carboxymethylcellulose wird mit Ethanol angeteigt. Dieser Natrium Carboxymethylcellulose-Suspension werden Wasser und Cardiospermum halicacabum Urtinktur (bzw. Extrakt) unter Rühren zugegeben, bis eine homogene Lösung (wässrige Phase) entsteht.
2. Das dünnflüssige Paraffin (Ölphase) wird in die wässrige Phase eingearbeitet, und diese Mischung wird mit einem Rotor-Stator-Homogenisator homogenisiert.

### Beispiel 5: Rezeptur einer erfindungsgemäßen celluloseetberstabilisierten Hamamelis-Urtinktur-haltigen O/W-Emulsion

### Inhaltsstoffe:

| | |
|---|---|
| Hypromellose Ph Eur | 2,0 % m/m |
| Hamamelis Ø HAB | 20,0 % m/m |
| Ethanol Ph Eur | 5,0 % m/m |
| Mittelkettige Triglyceride Ph Eur | 20,0 % m/m |
| Ger. Wasser Ph Eur | ad 100,0 % m/m |

### Zubereitung:

1. Die Hypromellose wird mit Ethanol angeteigt. Dieser Hypromellosesuspension werden Wasser und Hamamelisurtinktur unter Rühren zugegeben, bis eine homogene Lösung (wässrige Phase) entsteht.
2. Die mittelkettigen Triglyceride (Ölphase), werden in die wässrige Phase eingearbeitet, und diese Mischung wird mit einem Rotor-Stator-Homogenisator homogenisiert.

### Beispiel 6: Rezeptur einer erfindungsgemäßen celluloseetherstabilisierten Calendula-Urtinktur-haltigen O/W-Emulsion

### Inhaltsstoffe:

| | |
|---|---|
| Hypromellose Ph Eur | 2,0 % m/m |
| Calendula Ø HAB | 10,0 % m/m |
| Ethanol Ph Eur | 8,0 % m/m |
| Mittelkettige Triglyceride Ph Eur | 20,0 % m/m |
| Ger. Wasser Ph Eur | ad 100,0 % m/m |

### Zubereitung:

1. Die Hypromellose wird mit Ethanol angeteigt. Dieser Hypromellosesuspension werden Wasser und Calendulaurtinktur unter Rühren zugegeben, bis eine homogene Lösung (wässrige Phase) entsteht.
2. Die mittelkettigen Triglyceride (Ölphase), werden in die wässrige Phase eingearbeitet, und diese Mischung wird mit einem Rotor-Stator-Homogenisator

### Beispiel 7: Rezeptur einer erfindungsgemäßen celluloseetherstabilisierten Berberis-Urtinktur-haltigen O/W-Emulsion

### Inhaltsstoffe:

| | |
|---|---|
| Hypromellose Ph Eur | 2,0 % m/m |
| Berberis Ø HAB | 5,0 % m/m |
| Ethanol Ph Eur | 10,0 % m/m |
| Mittelkettige Triglyceride Ph Eur | 20,0 % m/m |
| Ger. Wasser Ph Eur | ad 100,0 % m/m |

### Zubereitung:

1. Die Hypromellose wird mit Ethanol angeteigt. Dieser Hypromellosesuspension werden Wasser und Berberisurtinktur unter Rühren zugegeben, bis eine homogene Lösung (wässrige Phase) entsteht.
2. Die mittelkettigen Triglyceride (Ölphase), werden in die wässrige Phase eingearbeitet, und diese Mischung wird mit einem Rotor-Stator-Homogenisator.

### Beispiel 8: Rezeptur einer erfindungsgemäßen celluloseetherstabilisierten Cardiospermum-Urtinktur-haltigen O/W-Emulsion

### Inhaltsstoffe:

| | |
|---|---|
| Hypromellose Ph Eur | 10,0 % m/m |
| Cardiospermum Ø HAB | 10,0 % m/m |
| Ethanol Ph Eur | 8,0 % m/m |
| Mittelkettige Triglyceride Ph Eur | 30,0 % m/m |
| Ger. Wasser Ph Eur | ad 100,0 % m/m |

### Zubereitung:

1. Die Hypromellose wird mit Ethanol angeteigt. Dieser Hypromellosesuspension werden Wasser und Cardiospermumurtinktur unter Rühren zugegeben, bis eine homogene Lösung (wässrige Phase) entsteht.
2. Die mittelkettigen Triglyceride (Ölphase), werden in die wässrige Phase eingearbeitet, und diese Mischung wird mit einem Rotor-Stator-Homogenisator.

### Beispiel 9: Rezeptur einer erfindungsgemäßen celluloseetherstabilisierten Cardiospermum-Urtinktur-haltigen O/W-Emulsion

### Inhaltsstoffe:

| | |
|---|---|
| Hypromellose Ph Eur | 5,0 % m/m |
| Cardiospermum Ø HAB | 5,0 % m/m |
| Ethanol Ph Eur | 20,0 % m/m |
| Mittelkettige Triglyceride Ph Eur | 40,0 % m/m |
| Ger. Wasser Ph Eur | ad 100,0 % m/m |

### Zubereitung:

1. Die Hypromellose wird mit Ethanol angeteigt. Dieser Hypromellosesuspension werden Wasser und Cardiospermumurtinktur unter Rühren zugegeben, bis eine homogene Lösung (wässrige Phase) entsteht.
2. Die mittelkettigen Triglyceride (Ölphase), werden in die wässrige Phase eingearbeitet, und diese Mischung wird mit einem Rotor-Stator-Homogenisator.

### Beispiel 10: Rezeptur einer erfindungsgemäßen celluloseetherstabilisierten Cardiospermum-Urtinktur-haltigen O/W-Emulsion

### Inhaltsstoffe:

| | |
|---|---|
| Hypromellose Ph Eur | 1,0 % m/m |
| Cardiospermum Ø HAB | 10,0 % m/m |
| Ethanol Ph Eur | 8,0 % m/m |
| Mittelkettige Triglyceride Ph Eur | 60,0 % m/m |
| Ger. Wasser Ph Eur | ad 100,0 % m/m |

### Zubereitung:

1. Die Hypromellose wird mit Ethanol angeteigt. Dieser Hypromellosesuspension werden Wasser und Cardiospermumurtinktur unter Rühren zugegeben, bis eine homogene Lösung (wässrige Phase) entsteht.
2. Die mittelkettigen Triglyceride (Ölphase), werden in die wässrige Phase eingearbeitet, und diese Mischung wird mit einem Rotor-Stator-Homogenisator.

## Patentansprüche

1. Öl-in-Wasser-Emulsionszubereitung mit einem Gehalt an einer alkoholhaltigen homöopathischen und/oder pflanzlichen Wirkstoffzubereitung,
**dadurch gekennzeichnet,**
**dass** die Emulsionszubereitung aus den Komponenten Öl, Wasser, Alkohol, homöopathischer und/oder pflanzlicher Wirkstoff und Celluloseether besteht.

2. Öl-in-Wasser-Emulsionszubereitung nach Anspruch 1,
**dadurch gekennzeichnet,**
**dass** der Gehalt an Celluloseether 0,1-10 Masseprozent beträgt.

3. Öl-in-Wasser-Emulsionszubereitung nach Anspruch 1 oder 2,
**dadurch gekennzeichnet,**
**dass** der Gehalt an alkoholhaltiger homöopathischer und/oder pflanzlicher Wirkstoffzubereitung 1-50 Masseprozent beträgt.

4. Öl-in-Wasser-Emulsionszubereitung nach einem der Ansprüche 1 bis 3,
**dadurch gekennzeichnet,**
**dass** der Ölgehalt 1 - 74 Masseprozent beträgt.

5. Öl-in-Wasser-Emulsionszubereitung nach einem der Ansprüche 1 bis 4,
**dadurch gekennzeichnet,**
**dass** als Celluloseether Hypromelose bzw. Methylhydroxypropylcellulose - einzeln oder in Kombination mit anderen Celluloseethern - eingesetzt ist.

6. Öl-in-Wasser-Emulsionszubereitung nach einem der Ansprüche 1 bis 5,
**dadurch gekennzeichnet,**
**dass** als Celluloseether Hydroxypropylcellulose - einzeln oder in Kombination mit anderen Celluloseethern - eingesetzt ist.

7. Öl-in-Wasser-Emulsionszubereitung nach einem der Ansprüche 1 bis 6,
**dadurch gekennzeichnet,**
**dass** als Celluloseether Methylcellulose - einzeln oder in Kombination mit anderen Celluloseethern - eingesetzt ist.

8. Öl-in-Wasser-Emulsionszubereitung nach einem der Ansprüche 1 bis 7,
**dadurch gekennzeichnet,**
**dass** als Celluloseether Methylhydroxyethylcellulose und/oder Hydroxyethylcellulose und/oder Natriumcarboxymethylcellulose eingesetzt ist.

9. Öl-in-Wasser-Emulsionszubereitung nach einem der Ansprüche 1 bis 8,
**dadurch gekennzeichnet,**
**dass** die Ölphase vollständig oder teilweise aus mittelkettigen Triglyceriden besteht.

10. Öl-in-Wasser-Emulsionszubereitung nach einem der Ansprüche 1 bis 9,
**dadurch gekennzeichnet,**
**dass** die Ölphase vollständig oder teilweise aus Erdnußöl besteht.

11. Öl-in-Wasser-Emulsionszubereitung nach einem der Ansprüche 1 bis 10,
**dadurch gekennzeichnet,**
**dass** die Ölphase vollständig oder teilweise aus Rizinusöl besteht.

12. Öl-in-Wasser-Emulsionszubereitung nach einem der Ansprüche 1 bis 11,
**dadurch gekennzeichnet,**
**dass** die Ölphase vollständig oder teilweise aus dünnflüssigem Paraffin besteht.

13. Öl-in-Wasser-Emulsionszubereitung nach einem der Ansprüche 1 bis 12,
**dadurch gekennzeichnet,**
**dass** die Ölphase vollständig oder teilweise aus dickflüssigem Paraffin besteht.

14. Öl-in-Wasser-Emulsionszubereitung nach einem der Ansprüche 1 bis 13,
**dadurch gekennzeichnet,**
**dass** die Emulsionszubereitung zusätzlich zu der alkoholhaltigen homöopathischen und/oder pflanzlichen Wirkstoffzubereitung einen Zusatz an 1-20 Masseprozent Ethanol aufweist.

15. Verfahren zur Herstellung einer celluloseetherstabilisierten Öl-in-Wasser-Emulsionszubereitung nach einem der Ansprüche 1 bis 14,
**dadurch gekennzeichnet,**
**dass** zunächst der Celluloseether in der alkoholisch-wässrigen Emulsionsphase gelöst wird und anschließend diese celluloseetherhaltige, alkoholisch-wässrige Emulsionsphase mit der Ölphase homogenisiert wird.

16. Verwendung celluloseetherstabilisierter Öl-in-Wasser-Emulsionszubereitung nach einem der Ansprüche 1 bis 15 zur Herstellung homöopathischer und/oder phytopharmazeutischer Arzneimittel.

## Claims

1. An oil-in-water emulsion preparation with a content of an alcohol-containing preparation of homeopathic and/or herbal active ingredients, **characterized in that** the emulsion preparation consists of the components oil, water, alcohol, homeopathic and/or herbal active ingredients and cellulose ether.

2. The oil-in-water emulsion preparation according to claim 1, **characterized in that** the amount of cellulose ether is 0,1 - 10 mass percent.

3. The oil-in-water emulsion preparation according to claim 1 or 2, **characterized in that** the amount of the alcohol-containing preparation of homeopathic and/or herbal active ingredients is 1 - 50 mass percent.

4. The oil-in-water emulsion preparation according to one of claims 1 to 3, **characterized in that** the amount of oil is 1 - 74 mass percent.

5. The oil-in-water emulsion preparation according to one of claims 1 to 4, **characterized in that** hypromellose or methylhydroxypropylcellulose is used as the cellulose ether - individually or together with other cellulose ethers.

6. The oil-in-water emulsion preparation according to one of claims 1 to 5, **characterized in that** hydroxypropylcellulose is used as the cellulose ether - individually or together with other cellulose ethers.

7. The oil-in-water emulsion preparation according to one of claims 1 to 6, **characterized in that** methylcellulose is used as the cellulose ether - individually or together with other cellulose ethers.

8. The oil-in-water emulsion preparation according to one of claims 1 to 7, **characterized in that** methylhydroxyethylcellulose and/or hydroxyethylcellulose and/or sodium carboxymethylcellulose are used as the cellulose ether.

9. The oil-in-water- emulsion preparation according to one of claims 1 to 8, **characterized in that** the oil phase consists completely or partially of medium-chain triglycerides.

10. The oil-in-water emulsion preparation according to one of claims I to 9, **characterized in that** the oil phase consists completely or partially of peanut oil.

11. The oil-in-water emulsion preparation according to one of claims 1 to 10, **characterized in that** the oil phase consists completely or partially of castor oil.

12. The oil-in-water emulsion preparation according to one of claims 1 to 11, **characterized in that** the oil phase consists completely or partially of highly-liquid paraffin.

13. The oil-in-water emulsion preparation according to one of claims 1 to 12, **characterized in that** the oil phase consists completely or partially of highly-viscous paraffin.

14. The oil-in-water emulsion preparation according to one of claims 1 to 13, **characterized in that** the emulsion preparation also contains 1 - 20 mass percent ethanol in addition to the alcohol-containing preparation of homeopathic and/or herbal active ingredients.

15. A procedure to prepare a cellulose-ether-stabilized oil-in-water emulsion preparation according to one of claims 1 to 14, **characterized in that** first the cellulose ether is dissolved in the alcoholic-aqueous emulsion phase, and then this cellulose-ether-containing, alcoholic-aqueous emulsion phase is homogenized with the oil phase.

16. The use of a cellulose-ether-stabilized, oil-in-water emulsion preparation according to one of claims 1 to 15 for preparation of homeopathic and/or phytopharmaceutical drugs.

## Revendications

1. Préparation à base d'une émulsion huile dans eau avec une teneur en une préparation de principes actifs homéopathiques et / ou végétaux contenant de l'alcool, **caractérisée en ce que** la préparation à base d'une émulsion est composée à base d'huile, d'eau, d'alcool, de principes actifs homéopathiques et / ou végétaux et d'éther de cellulose.

2. Préparation à base d'une émulsion huile dans eau selon la revendication 1, **caractérisé en ce que** la teneur en éther de cellulose est comprise entre 0,1 et 10 pour cent en masse.

3. Préparation à base d'une émulsion huile dans eau selon la revendication 1 ou 2, **caractérisée en ce que** la teneur en préparation de principes actifs homéopathiques et / ou végétaux contenant de l'alcool est comprise entre 1 et 50 pour cent en masse.

4. Préparation à base d'une émulsion huile dans eau selon l'une des revendications 1 à 3, **caractérisée en ce que** la teneur en huile est comprise entre 1 et 74 pour cent en masse.

5. Préparation à base d'une émulsion huile dans eau selon l'une des revendications 1 à 4, **caractérisée en ce qu'**on utilise comme éther de cellulose de l'hypromellose ou de la méthylhydroxypropylcellulose - seule ou en combinaison avec d'autres éthers de cellulose.

6. Préparation à base d'une émulsion huile dans eau selon l'une des revendications 1 à 5, **caractérisée en ce qu'**on utilise comme éther de cellulose de l'hydroxypropylcellulose - seule ou en combinaison avec d'autres éthers de cellulose.

7. Préparation à base d'une émulsion huile dans eau selon l'une des revendications 1 à 6, **caractérisée en ce qu'**on utilise comme éther de cellulose de la méthylcellulose - seule ou en combinaison avec d'autres éthers de cellulose.

8. Préparation à base d'une émulsion huile dans eau selon l'une des revendications 1 à 7, **caractérisée en ce qu'**on utilise comme éther de cellulose de la méthylhydroxyéthylcellulose et / ou de l'hydroxyéthylcellulose et / ou de la carboxyméthylcellulose sodique.

9. Préparation à base d'une émulsion huile dans eau selon l'une des revendications 1 à 8, **caractérisée en ce que** la phase huileuse se compose entièrement ou en partie de triglycérides à chaîne moyenne.

10. Préparation à base d'une émulsion huile dans eau selon l'une des revendications 1 à 9, **caractérisée en ce que** la phase huileuse se compose entièrement ou en partie d'huile d'arachide.

11. Préparation à base d'une émulsion huile dans eau selon l'une des revendications 1 à 10, **caractérisée en ce que** la phase huileuse se compose entièrement ou en partie d'huile de ricin.

12. Préparation à base d'une émulsion huile dans eau selon l'une des revendications 1 à 11, **caractérisée en ce que** la phase huileuse se compose entièrement ou en partie de paraffine fluide.

13. Préparation à base d'une émulsion huile dans eau selon l'une des revendications 1 à 12, **caractérisée en ce que** la phase huileuse se compose entièrement ou en partie de paraffine épaisse.

14. Préparation à base d'une émulsion huile dans eau selon l'une des revendications 1 à 13, **caractérisée en ce qu'**outre la préparation de principes actifs homéopathiques et / ou végétaux contenant de l'alcool, la préparation à base d'une émulsion contient en plus un ajout d'éthanol de 1 à 20 pour cent en masse.

15. Procédé pour la fabrication d'une préparation à base d'une émulsion huile dans eau stabilisée à l'aide d'éther de cellulose selon l'une des revendications 1 à 14, **caractérisé en ce que** l'éther de cellulose est tout d'abord solubilisé dans la phase d'émulsion eau-alcool, puis cette phase d'émulsion eau-alcool contenant l'éther de cellulose est homogénéisée avec la phase huileuse.

16. Utilisation d'une préparation à base d'une émulsion huile dans eau stabilisée à l'aide d'éther de cellulose selon l'une des revendications 1 à 15 pour la fabrication de médicaments homéopathiques et / ou phytopharmaceutiques.
